# EUROPEAN PATENT APPLICATION

(11) **EP 4 710 999 A1**
(43) Date of publication of application: **18.03.2026**
(21) Application number: 24200021.4
(22) Date of filing: 12.09.2024
(51) Int. Cl.: A61N 5/10, A61B 6/03

(54) **METHOD FOR DETERMINING AN OVERDOSING IN RADIATION THERAPY**

(71) Applicant: Siemens Healthineers AG, 91301 Forchheim (DE)
(72) Inventor: Holbrook, Matthew, Pennington, 08534 (US); Santhanam, Anand, Monmouth Junction, 08852 (US)
(74) Representative: Siemens Healthineers Patent Attorneys

(57) **Abstract**

The invention relates to a system and a method for determining an overdosing in radiation therapy, comprising the following steps:
- Acquiring image data by a photon counting imaging device,
- Applying a reconstruction algorithm to the image data, wherein reconstructed image data are generated,
- Applying an analysis algorithm to analysis input data and generating analysis data, wherein the analysis input data comprise the reconstructed image data, wherein the analysis algorithm is configured to detect an overdosing based on the analysis input data, wherein the analysis data comprise the detected overdosing and/or an information about the overdosing detected by the analysis algorithm, and
- Providing the analysis information.

## Description

This invention relates to the field of radiation therapy, especially imaging methods for planning and monitoring of radiation therapies. In particular, the invention relates to a method and a system for detecting overdosed tissues in radiation therapy, especially using photon counting computed tomography (PC-CT).

Radiation therapy (RT) is an important treatment method for various types of cancer. In this method, the tumor region is irradiated with ionizing radiation to kill or inhibit the growth of cancer cells. To perform an effective and safe RT, it is necessary to image the treatment region beforehand to determine the geometric relationship of lesions and surrounding tissue. Moreover, the treatment region must be monitored regularly to assess the treatment efficiency and adjust the treatment plans accordingly. The health of the surrounding tissue is of great importance, as this tissue often reacts sensitively to radiation and can be easily damaged by overdosing. Unfortunately, it is often not possible with the current imaging methods to detect damage to healthy tissue by overdosing early, before it becomes chronic. The early detection of tissue overdosing is especially dependent on the contrast between soft tissues.

The state of the art in imaging methods for RT planning and monitoring therefore has some disadvantages that can affect the quality and safety of RT. On the one hand, computed tomography (CT) is the preferred modality for RT planning, as it provides excellent resolution and standardized image values (HU, Hounsfield units) that can be converted into attenuation coefficients. The RT planning using CT is performed before the first dose fraction of the patient. The most common form of follow-up imaging for RT is the use of cone-beam CT (CBCT) scanners, which have a better form factor for operation in RT rooms. However, these systems produce images that are less sharp and more prone to sampling and motion artifacts. Moreover, the standardized HU values are less reliable than with a conventional CT scanner.

With this technology, overdosing is assessed by imaging the treatment region and looking for signs of damage to healthy tissue. The problems with image quality directly affect the ability to detect problems before they become chronic.

It is therefore an object of the present invention to provide a method and a system for detecting overdosed tissues in radiation therapy that enable improved image quality and increased soft tissue contrast.

Independent of the grammatical term usage, individuals with male, female or other gender identities are included within the term.

The invention proposes to use photon counting based imaging device, especially a photon counting computed tomography (PC-CT), as an imaging modality for RT planning and monitoring. Photon counting imaging, especially PC-CT is a novel technology that measures the energy of the incident photons and enables spectral imaging. The PC-CT offers several advantages over conventional CT, such as higher resolution, lower radiation dose, better material discrimination, and reduction of scatter and noise. The PC-CT can thus improve the image quality and the soft tissue contrast and enable a more accurate detection of overdosed tissues in RT.

The present invention relates to a method for determining an overdosing in radiation therapy, which can be used to monitor and adjust the radiation dose delivered to a target tissue or organ.

The method comprises the following steps:
- Acquiring image data by a photon counting imaging device,
- Applying a reconstruction algorithm to the image data, wherein reconstructed image data are generated,
- Applying an analysis algorithm to analysis input data and generating analysis data, wherein the analysis input data comprise the reconstructed image data, wherein the analysis algorithm is configured to detect an overdosing based on the analysis input data, wherein the analysis data comprise the detected overdosing and/or an information about the overdosing detected by the analysis algorithm, and
- Providing the analysis information.

Acquiring image data by a photon counting imaging device may involve using a device that can detect and count individual photons of radiation that pass through or are scattered by the tissue or organ of interest. The device can be, for example, a photon counting detector, a photon counting computed tomography (CT) scanner, a photon counting positron emission tomography (PET) scanner, or any other suitable device that can acquire image data based on photon counting. The image data can represent the spatial distribution, energy spectrum, or attenuation of the photons in the tissue or organ. The image data may comprise and/or show a region of interested, especially a target, e.g., a tumor, and/or surrounding tissue, structures and/or organs.

Applying a reconstruction algorithm to the image data may involve using a mathematical or computational method to reconstruct an image or a volume from the acquired image data. The reconstruction algorithm can be, for example, a filtered back-projection algorithm, an iterative reconstruction algorithm, a maximum likelihood algorithm, a maximum a posteriori algorithm, or any other suitable algorithm that can generate reconstructed image data from the photon counting image data. The reconstructed image data can provide information about the density, composition, or structure of the tissue or organ.

Applying an analysis algorithm to analysis input data and generating analysis data may involve using a method to analyze the reconstructed image data and to identify any regions or areas where the radiation dose exceeds a predetermined threshold or limit. The analysis algorithm can be, for example, a segmentation algorithm, a classification algorithm, a clustering algorithm, a thresholding algorithm, a statistical algorithm, a machine learning algorithm, or any other suitable algorithm that can detect an overdosing based on the reconstructed image data. The analysis data can include the location, size, shape, or extent of the overdosing, or any other information that can be useful for assessing the severity or impact of the overdosing.

Providing the analysis information may involve presenting or displaying the analysis data to a user, such as a radiation therapist, a radiologist, a physician, or a patient. The analysis information can be provided, for example, as an image, a graph, a table, a text, a sound, a vibration, or any other suitable form of communication. The analysis information can help the user to evaluate the overdosing and to take appropriate actions, such as modifying the radiation plan, adjusting the radiation parameters, or applying a corrective treatment. Providing the analysis information may comprise providing the analysis information to a treatment planning system or to a treatment planning method, for example to adapt and/or verify a treatment plan.

In one embodiment, the method can be used to determine an overdosing in radiation therapy for cancer treatment. The photon counting imaging device can acquire image data of the tumor and the surrounding healthy tissue before, during, or after the radiation therapy. The reconstruction algorithm can generate reconstructed image data that show the contrast or difference between the tumor and the healthy tissue. The analysis algorithm can detect any regions where the radiation dose delivered to the healthy tissue is higher than the radiation dose delivered to the tumor, indicating an overdosing. The analysis information can alert the user about the overdosing and suggest possible ways to reduce or prevent it.

In another embodiment, the method can be used to determine an overdosing in radiation therapy for brain disorders, such as epilepsy, Parkinson's disease, or Alzheimer's disease. The photon counting imaging device can acquire image data of the brain and the different brain regions or structures involved in the disorder. The reconstruction algorithm can generate reconstructed image data that show the functional or metabolic activity of the brain regions or structures. The analysis algorithm can detect any regions where the radiation dose delivered to the brain regions or structures is higher than the radiation dose required to achieve the desired therapeutic effect, indicating an overdosing. The analysis information can inform the user about the overdosing and recommend possible ways to optimize or improve the radiation therapy. Interactions and technical effects:
The method according to the invention can provide a fast and accurate way to determine an overdosing in radiation therapy, which can improve the quality and safety of the radiation therapy. The method can take advantage of the high sensitivity, resolution, and dynamic range of the photon counting imaging device, which can capture the radiation dose distribution and the tissue or organ characteristics in detail. The method can also use the reconstruction algorithm and the analysis algorithm to process and interpret the image data, which can enhance the detection and quantification of the overdosing. The method can further provide the analysis information to the user, which can facilitate the monitoring and adjustment of the radiation therapy.

The method according to the invention can also provide a flexible and adaptable way to determine an overdosing in radiation therapy, which can suit different applications and scenarios. The method can be compatible with various types of photons counting imaging devices, reconstruction algorithms, and analysis algorithms, which can be selected or customized according to the specific needs or preferences of the user. The method can also be applicable to different types of radiation therapy, such as external beam radiation therapy, internal radiation therapy, or proton therapy, and to different types of tissue or organ, such as lung, liver, breast, prostate, or brain.

In an preferred embodiment of the invention, the reconstruction algorithm comprises a multi-channel reconstruction algorithm and/or an artifact reduction algorithm, wherein the step of applying the analysis algorithm comprises generating multi-channel reconstructed image data by applying the multi-channel reconstruction algorithm to the image data and/or generating artifact reduced image data by applying the artifact reduction algorithm to the image data, wherein reconstructed image data comprise and/or are based on the multi-channel reconstructed image data and/or the artifact reduced image data. The reconstruction algorithm can comprise a multi-channel reconstruction algorithm and/or an artifact reduction algorithm, which can improve the quality and accuracy of the reconstructed image data.

The multi-channel reconstruction algorithm can be an algorithm that can exploit the energy information of the photons to generate multi-channel reconstructed image data, which can represent different energy ranges or windows of the photons. The multi-channel reconstruction algorithm can separate the image data into different channels according to the energy thresholds or bins, and then apply a reconstruction method, such as filtered back projection or iterative reconstruction, to each channel. The multi-channel reconstruction algorithm can enhance the contrast and resolution of the reconstructed image data and can also reduce the noise and scatter effects.

The artifact reduction algorithm is especially an algorithm that can reduce or eliminate the artifacts that may occur in the image data, such as beam hardening, photon starvation, or metal artifacts. The artifact reduction algorithm can apply various techniques, such as preprocessing, postprocessing, or correction methods, to the image data to generate artifact reduced image data, which can have less distortion and more accurate representation of the radiation dose distribution and the tissue or organ characteristics.

The step of applying the analysis algorithm comprises especially generating multi-channel reconstructed image data by applying the multi-channel reconstruction algorithm to the image data and/or generating artifact reduced image data by applying the artifact reduction algorithm to the image data. The analysis algorithm can be an algorithm that can analyze the reconstructed image data to detect and quantify an overdosing in radiation therapy. The analysis algorithm can use various methods, such as segmentation, classification, or regression, to extract and compare the features of the reconstructed image data, such as the dose distribution, the tissue or organ density, or the contrast-to-noise ratio, and to determine whether the features indicate an overdosing or not. The analysis algorithm can especially also output the analysis information, such as the location, size, or severity of the overdosing, to the user. The reconstructed image data comprise and/or are based on the multi-channel reconstructed image data and/or the artifact reduced image data, which can provide more information and accuracy for the analysis algorithm.

An example of an embodiment of the method according to the invention is as follows:
A photon counting imaging device is used to acquire image data of a patient who undergoes radiation therapy for lung cancer. The photon counting imaging device can measure the energy and number of the photons in the radiation beam that passes through the patient's chest. The image data can represent the radiation dose distribution and the lung characteristics of the patient. A reconstruction algorithm is applied to the image data to reconstruct the image data. The reconstruction algorithm comprises a multi-channel reconstruction algorithm and an artifact reduction algorithm. The multi-channel reconstruction algorithm can separate the image data into four channels according to the energy ranges of 20-40 keV, 40-60 keV, 60-80 keV, and 80-100 keV, and then apply an iterative reconstruction method to each channel. The multi-channel reconstruction algorithm can generate multi-channel reconstructed image data, which can show the different energy levels of the photons and the different contrast and resolution of the lung tissue. The artifact reduction algorithm can apply a preprocessing technique to the image data to correct the beam hardening effect, which can cause the image data to have lower intensity and higher attenuation than expected. The artifact reduction algorithm can generate artifact reduced image data, which can have less distortion and more accurate dose distribution.

An analysis algorithm is applied to the reconstructed image data to determine an overdosing in radiation therapy. The analysis algorithm can use a segmentation method to segment the reconstructed image data into different regions, such as the lung, the tumor, and the surrounding tissue. The analysis algorithm can then use a classification method to classify each region according to the dose distribution and the lung density, and to identify the regions that have an overdosing, which can be defined as a dose that exceeds the prescribed dose by more than 10%. The analysis algorithm can also use a regression method to estimate the size and severity of the overdosing, which can be measured by the percentage of the dose deviation and the volume of the affected region. The analysis algorithm can output the analysis information, such as the location, size, and severity of the overdosing, to the user, who can monitor and adjust the radiation therapy accordingly.

In a preferred embodiment of the invention, the method comprises an auto contouring step. The auto contouring step is preferably a step between the step of applying the reconstruction algorithm and the step of applying the analysis algorithm. The auto contouring step comprises generating auto contoured image data by applying an auto contouring algorithm to the reconstructed image data, wherein the analysis input data comprise the auto contoured image data, wherein the analysis algorithm is configured to detect the overdosing based on the auto contoured image data.

The auto contouring step is preferably a step between the step of applying the reconstruction algorithm and the step of applying the analysis algorithm. The auto contouring step comprises generating auto contoured image data by applying an auto contouring algorithm to the reconstructed image data. The analysis input data comprise the auto contoured image data, wherein the analysis algorithm is configured to detect the overdosing based on the auto contoured image data.

The auto contouring step can be any step that can generate auto contoured image data by applying an auto contouring algorithm to the reconstructed image data. For example, the auto contouring step can be a step that can use an edge detection method, a region growing method, or a level set method to detect the boundaries of the regions of interest in the reconstructed image data. The auto contouring step can generate auto contoured image data, which can have more defined and accurate contours of the regions of interest, such as the lung, the tumor, and the surrounding tissue. The auto contoured image data can be used as the input data for the analysis algorithm, which can detect the overdosing based on the auto contoured image data.

In a preferred embodiment of the invention, the method comprises derivative image data step. The derivative image data step is preferably a step between the step of applying the reconstruction algorithm and the step of applying the analysis algorithm. The derivative image data step comprises generating derivative image data by applying an derivative image data algorithm to the reconstructed image data, wherein the analysis input data comprise the derivative image data, wherein the analysis algorithm is configured to detect the overdosing based on the derivative image data.

Preferably, the derivative image data algorithm is configured to generate a derivative map, a water/Ca map, a CS/PE map and/or a mono-energetic map based on the reconstructed image data, wherein the derivative image data comprise the derivative map, water/Ca map, CS/PE map and/or a mono-energetic map.

In a preferred embodiment of the invention, the method comprises a derivative image data step. The derivative image data step is preferably a step between the step of applying the reconstruction algorithm and the step of applying the analysis algorithm. The derivative image data step comprises generating derivative image data by applying a derivative image data algorithm to the reconstructed image data, wherein the analysis input data comprise the derivative image data, wherein the analysis algorithm is configured to detect the overdosing based on the derivative image data.

The derivative image data step can be any step that can generate derivative image data by applying a derivative image data algorithm to the reconstructed image data. For example, the derivative image data step can be a step that can use a dual-energy method, a material decomposition method, or a spectral imaging method to generate different types of derivative image data based on the reconstructed image data. The derivative image data step can generate derivative image data, which can have more information and contrast of the regions of interest, such as the lung, the tumor, and the surrounding tissue. The derivative image data can be used as the input data for the analysis algorithm, which can detect the overdosing based on the derivative image data.

Preferably, the derivative image data algorithm is configured to generate a derivative map, a water/Ca map, a CS/PE map and/or a mono-energetic map based on the reconstructed image data, wherein the derivative image data comprise the derivative map, water/Ca map, CS/PE map and/or a mono-energetic map. The derivative map can show the spatial distribution of the derivative image data, such as the dual-energy index, the material density, or the spectral intensity. The water/Ca map can show the relative proportions of water and calcium in the regions of interest. The CS/PE map can show the relative proportions of contrast medium and soft tissue in the regions of interest. The mono-energetic map can show the reconstructed image data at a specific photon energy level. The derivative image data can provide more information and contrast for the analysis algorithm to detect the overdosing.

An optional embodiment of the invention comprises a radiomic analysis step, wherein the radiomic analysis step comprises generating radiomic analysis data by applying an radiomic algorithm to the image data, the reconstructed image data, the auto contoured image data and/or the derivative image data, wherein the analysis input data comprise the radiomic analysis data, wherein the analysis algorithm is configured to detect the overdosing based on the radiomic analysis data.

The radiomic analysis step is preferably a step between the step of applying the reconstruction algorithm and the step of applying the analysis algorithm. The radiomic analysis step comprises generating radiomic analysis data by applying a radiomic algorithm to the image data, the reconstructed image data, the automatically contoured image data and/or the derived image data, wherein the analysis input data comprise the radiomic analysis data, wherein the analysis algorithm is configured to detect the overdosing based on the radiomic analysis data. The radiomic algorithm is configured to extract radiomic features from the image data, the reconstructed image data, the automatically contoured image data and/or the derived image data, which can describe the texture, the shape, the intensity and/or the heterogeneity of the regions of interest. The radiomic analysis data can comprise the radiomic features and/or statistical parameters, which are calculated from the radiomic features. The radiomic analysis data can provide more information and correlations about the regions of interest, which are relevant for the detection of the overdosing.

An optional embodiment of the invention comprises a attenuation coefficient step, wherein the attenuation coefficient step comprises generating attenuation coefficients by applying an attenuation coefficient determination algorithm to the image data, the reconstructed image data, the auto contoured image data and/or the derivative image data, wherein the analysis input data comprise the attenuation coefficients, wherein the analysis algorithm is configured to detect the overdosing based on the attenuation coefficients.

An optional embodiment of the invention comprises an attenuation coefficient step, wherein the attenuation coefficient step comprises generating attenuation coefficients by applying an attenuation coefficient determination algorithm to the image data, the reconstructed image data, the auto contoured image data and/or the derivative image data. The attenuation coefficients are numerical values that indicate how much the image data are attenuated by the tissue or material that they pass through. For example, bone has a higher attenuation coefficient than soft tissue, and air has a lower attenuation coefficient than water. The analysis input data comprise the attenuation coefficients, wherein the analysis algorithm is configured to detect the overdosing based on the attenuation coefficients. The overdosing refers to the situation where the radiation dose delivered to the target tissue or organ is higher than the prescribed dose, which can cause adverse effects such as tissue damage or necrosis.

Preferably, the step of acquiring the image data comprises acquiring image data based on photon counting computed tomography (PCCT). PCCT is an advanced imaging modality that can measure the energy of the photons that pass through the patient's body and produce images with higher contrast and lower noise than conventional computed tomography (CT). The image data are especially acquired after a radiotherapy treatment and/or between fractions of a radiotherapy treatment, in order to monitor the changes in the tissue or organ that received the radiation dose. The image data are especially acquired when the patient is positioned for radiotherapy, so that the alignment and the shape of the target region can be verified and adjusted if necessary.

Optionally, the step providing the analysis information comprises providing the analysis information to a treatment planning algorithm, wherein the treatment planning algorithm is configured to adapt a treatment plan based on the analysis information, wherein the treatment plan is provided to a radiotherapy device to deliver the radiotherapy according to the provided treatment plan.

Optionally, the step of providing the analysis information to a treatment planning algorithm involves sending the data about the detected overdosing to a software program that can modify the parameters of the radiation therapy, such as the dose, the beam shape, the angle, the duration, or the frequency. The treatment planning algorithm can adjust the treatment plan based on the analysis information, in order to reduce the risk of further overdosing or to compensate for the underdosing in other regions. The modified treatment plan is then delivered to a radiotherapy device, such as a linear accelerator, a gamma knife, or a proton beam therapy machine, that can execute the radiation therapy according to the provided treatment plan. For example, if the analysis information indicates that the target tissue has received too much dose in one fraction, the treatment planning algorithm can lower the dose for the next fraction or change the beam shape to avoid the overdosed region.

The invention is also related to a system for determining an overdosing in radiation therapy, comprising:
- a photon counting imaging device configured to acquire image data,
- a reconstruction module configured to apply a reconstruction algorithm to the image data and generate reconstructed image data,
- an analysis module configured to apply an analysis algorithm to analysis input data and generate analysis data, wherein the analysis input data comprise the reconstructed image data, wherein the analysis algorithm is configured to detect an overdosing based on the analysis input data, wherein the analysis data comprise the detected overdosing and/or an information about the overdosing detected by the analysis algorithm, and
- a providing module configured to provide the analysis information.

Preferably, the photon counting imaging device is configured as a photon counting cone beam computed tomography (PC CBCT) device and/or comprises a flat panel photon counting detector.

Particularly, the system further comprises a radiotherapy treatment unit configured to deliver radiation therapy to a patient based on the analysis data.

The photon counting imaging device is a device that counts the number of photons that reach a detector after passing through an object, such as a patient. The photon counting imaging device may be a photon counting cone beam computed tomography (PC CBCT) device that uses a cone-shaped beam of X-rays and a flat panel photon counting detector to obtain the image data. Alternatively, the photon counting imaging device may use other types of X-ray sources and detectors. The photon counting imaging device may be implemented as a hardware device or a software module, or a combination thereof.

The reconstruction module is a module that processes the image data acquired by the photon counting imaging device and reconstructs them into a cross-sectional image of the patient. The reconstruction module may use various algorithms and techniques to perform the reconstruction, such as filtered back-projection, iterative reconstruction, or statistical reconstruction. The reconstruction module may be implemented as a hardware device or a software module, or a combination thereof. The reconstruction module may be connected to the photon counting imaging device via a wired or wireless connection or may be integrated with the photon counting imaging device.

The analysis module is a module that analyzes the reconstructed image data and detects an overdosing in radiation therapy based on predetermined criteria. The analysis module may use various methods and parameters to perform the analysis, such as comparing the reconstructed image data with a reference image, measuring the dose distribution, or identifying the regions of interest. The analysis module may generate analysis data that include the detected overdosing and/or an information about the overdosing, such as the location, the extent, the severity, or the cause of the overdosing. The analysis module may be implemented as a hardware device or a software module, or a combination thereof. The analysis module may be connected to the reconstruction module via a wired or wireless connection or may be integrated with the reconstruction module.

The communication module is a module that provides the analysis data to a user interface and/or a radiotherapy treatment unit. The communication module may use various protocols and formats to transmit the analysis data, such as TCP/IP, HTTP, XML, or JSON. The communication module may be implemented as a hardware device or a software module, or a combination thereof. The communication module may be connected to the analysis module via a wired or wireless connection or may be integrated with the analysis module.

The system may further comprise a radiotherapy treatment unit that delivers radiation therapy to the patient based on the analysis data. The radiotherapy treatment unit may include a linear accelerator (linac) that generates high-energy X-rays for the radiation therapy. The radiotherapy treatment unit may adjust the radiation dose, the beam angle, the beam shape, or the beam position based on the analysis data to avoid or reduce the overdosing. The radiotherapy treatment unit may be connected to the communication module via a wired or wireless connection or may be integrated with the communication module.

In addition or alternatively, features of different categories, such as process/method features or system features, can be interchangeable with each other. This means that a process/method feature can also be understood as a system feature and vice versa.

Further details, examples, advantages and effects of the present invention result from the following figures and their description. Thereby, the figures show:
Figure 1 a flowchart of a method for determining an overdosing in radiation therapy;
Figure 2 an example of a system for determining an overdosing in radiation therapy.

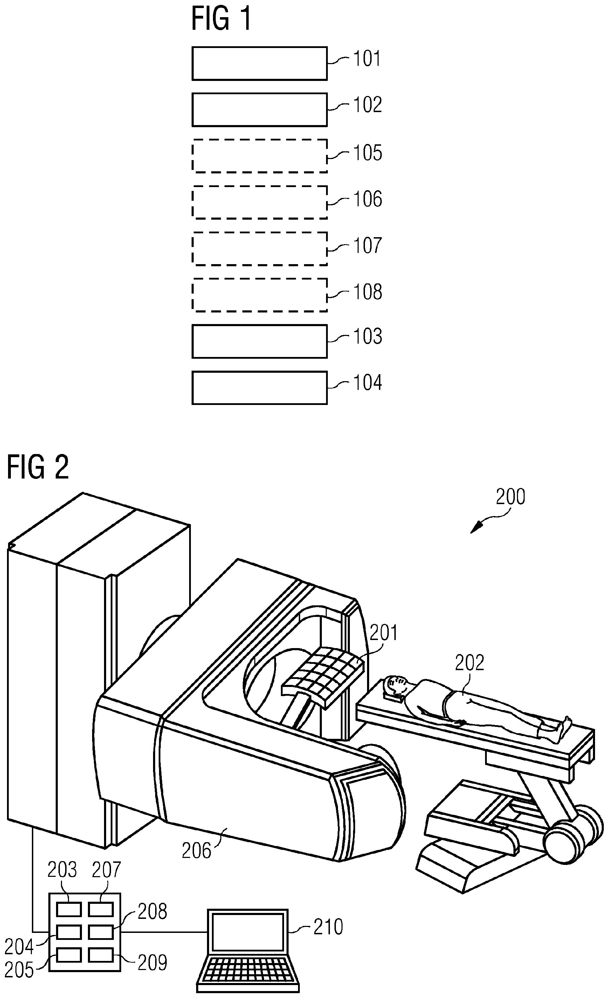

Figure 1 shows a flowchart of an exemplary method for determining an overdosing in radiation therapy

The method comprises the following steps:
- Step 101: Acquiring image data by a photon counting imaging device. For example, the photon counting imaging device can acquire CT images of a patient's lung and surrounding tissues, where the lung has a tumor that needs to be treated with radiation therapy.
- Step 102: Applying a reconstruction algorithm to the image data, wherein reconstructed image data are generated. For example, the reconstruction algorithm can generate reconstructed image data that have higher spatial resolution, lower noise, and lower radiation dose than conventional CT images.
- Step 103: Applying an analysis algorithm to analysis input data and generating analysis data, wherein the analysis input data comprise the reconstructed image data, wherein the analysis algorithm is configured to detect an overdosing based on the analysis input data, wherein the analysis data comprise the detected overdosing and/or an information about the overdosing detected by the analysis algorithm, and
- For example, the analysis algorithm can detect an overdosing by comparing the reconstructed image data with a reference image data that represents the desired radiation dose distribution for the lung tumor. The analysis data can include the location, size, and severity of the overdosing, as well as the potential effects on the normal tissues and organs.
- Step 104: Providing the analysis information.

For example, the analysis information can be provided to a treatment planning algorithm, a radiation oncologist, or a radiologist, who can use the analysis information to adjust the radiation therapy plan, monitor the treatment progress, or evaluate the treatment outcome.

In the exemplary method, the reconstruction algorithm comprises a multi-channel reconstruction algorithm and/or an artifact reduction algorithm, wherein the step of applying the analysis algorithm comprises generating multi-channel reconstructed image data by applying the multi-channel reconstruction algorithm to the image data and/or generating artifact reduced image data by applying the artifact reduction algorithm to the image data, wherein reconstructed image data comprise and/or are based on the multi-channel reconstructed image data and/or the artifact reduced image data.

For example, the multi-channel reconstruction algorithm can generate multi-channel reconstructed image data that contain spectral information of the photon counting imaging device, which can be used to differentiate different materials and tissues in the image. The artifact reduction algorithm can generate artifact reduced image data that remove or reduce the effects of beam hardening, scatter, or motion artifacts in the image.

In the exemplary method, the method further comprises an auto contouring step 105, wherein the auto contouring step comprises generating auto contoured image data by applying an auto contouring algorithm to the reconstructed image data, wherein the analysis input data comprise the auto contoured image data, wherein the analysis algorithm is configured to detect the overdosing based on the auto contoured image data.

For example, the auto contouring algorithm can generate auto contoured image data that automatically segment and label the lung tumor and the surrounding organs at risk in the reconstructed image data, which can facilitate the detection of overdosing and the evaluation of the radiation therapy plan.

In the exemplary method, the method further comprises a derivative image data step 106, wherein the derivative image data step comprises generating derivative image data by applying a derivative image data algorithm to the reconstructed image data, wherein the analysis input data comprise the derivative image data, wherein the analysis algorithm is configured to detect the overdosing based on the derivative image data. For example, the derivative image data algorithm can generate derivative image data that provide additional information about the physical and chemical properties of the tissues and materials in the reconstructed image data, such as the effective atomic number, the electron density, the water content, or the calcium content. The derivative image data can enhance the contrast and the accuracy of the overdosing detection.

In the exemplary method, the method further comprises a radiomic analysis step 107, wherein the radiomic analysis step comprises generating radiomic analysis data by applying a radiomic algorithm to the image data, the reconstructed image data, the auto contoured image data and/or the derivative image data, wherein the analysis input data comprise the radiomic analysis data, wherein the analysis algorithm is configured to detect the overdosing based on the radiomic analysis data. In the exemplary method, the analysis algorithm is configured to detect overdosing based on a contrast and/or contrast between tissues and/or structures.

In the exemplary method, the method further comprises an attenuation coefficient step 108, wherein the attenuation coefficient step comprises generating attenuation coefficients by applying an attenuation coefficient determination algorithm to the image data, the reconstructed image data, the auto contoured image data and/or the derivative image data, wherein the analysis input data comprise the attenuation coefficients, wherein the analysis algorithm is configured to detect the overdosing based on the attenuation coefficients.

In the exemplary method, the step 104 providing the analysis information comprises providing the analysis information to a treatment planning algorithm, wherein the treatment planning algorithm is configured to adapt a treatment plan based on the analysis information, wherein the treatment plan is provided to a radiotherapy device to deliver the radiotherapy according to the provided treatment plan.

Figure 2 shows a schematic diagram of an exemplary system 200 for determining an overdosing in radiation therapy.

The system 200 comprises:
- A photon counting imaging device 201, configured to acquire image data of a patient 202 undergoing radiation therapy,
- A reconstruction module 203, configured to apply a reconstruction algorithm to the image data, wherein reconstructed image data are generated,
- An analysis module 204, configured to apply an analysis algorithm to analysis input data and generate analysis data, wherein the analysis input data comprise the reconstructed image data, wherein the analysis algorithm is configured to detect an overdosing based on the analysis input data, wherein the analysis data comprise the detected overdosing and/or an information about the overdosing detected by the analysis algorithm, and
- A providing module 205 for providing the analysis information to a user, e.g. via an Interface 210, and/or to a treatment planning module, configured to receive the analysis information from the analysis module 204 and adapt a treatment plan based on the analysis information, wherein the treatment plan is provided to a radiotherapy unit 206 to deliver the radiotherapy according to the provided treatment plan.

The reconstruction module 203 may comprise a multi-channel reconstruction module and/or an artifact reduction module, wherein the multi-channel reconstruction module is configured to generate multi-channel reconstructed image data by applying a multi-channel reconstruction algorithm to the image data and/or the artifact reduction module is configured to generate artifact reduced image data by applying an artifact reduction algorithm to the image data, wherein reconstructed image data comprise and/or are based on the multi-channel reconstructed image data and/or the artifact reduced image data.

The system 200 may further comprise an auto contouring module 207, wherein the auto contouring module is configured to generate auto contoured image data by applying an auto contouring algorithm to the reconstructed image data, wherein the analysis input data comprise the auto contoured image data, wherein the analysis algorithm is configured to detect the overdosing based on the auto contoured image data.

The system 200 may further comprise a derivative image data module 208, wherein the derivative image data module is configured to generate derivative image data by applying a derivative image data algorithm to the reconstructed image data, wherein the analysis input data comprise the derivative image data, wherein the analysis algorithm is configured to detect the overdosing based on the derivative image data.

The derivative image data module may be configured to generate a derivative map, a water/Ca map, a CS/PE map and/or a mono-energetic map based on the reconstructed image data, wherein the derivative image data comprise the derivative map, water/Ca map, CS/PE map and/or a mono-energetic map.

The system 200 may further comprise a radiomic analysis module 209, wherein the radiomic analysis module is configured to generate radiomic analysis data by applying a radiomic algorithm to the image data, the reconstructed image data, the auto contoured image data and/or the derivative image data, wherein the analysis input data comprise the radiomic analysis data, wherein the analysis algorithm is configured to detect the overdosing based on the radiomic analysis data.

The analysis module 204 may be configured to detect overdosing based on a contrast and/or contrast between tissues and/or structures.

The system 200 may further comprise an attenuation coefficient module, wherein the attenuation coefficient module is configured to generate attenuation coefficients by applying an attenuation coefficient determination algorithm to the image data, the reconstructed image data, the auto contoured image data and/or the derivative image data, wherein the analysis input data comprise the attenuation coefficients, wherein the analysis algorithm is configured to detect the overdosing based on the attenuation coefficients.

The system 200 may also comprise an interface 210 for a user, e.g. comprising a monitor, to show the user the provided analysis information, such that the user can react on detected overdosing during the radiation therapy and for example stop or adapt the treatment manually.

## Claims

1. A method for determining an overdosing in radiation therapy, comprising the following steps:
- Acquiring image data (101) by a photon counting imaging device,
- Applying a reconstruction algorithm (102) to the image data, wherein reconstructed image data are generated,
- Applying an analysis algorithm to analysis (103) input data and generating analysis data, wherein the analysis input data comprise the reconstructed image data, wherein the analysis algorithm is configured to detect an overdosing based on the analysis input data, wherein the analysis data comprise the detected overdosing and/or an information about the overdosing detected by the analysis algorithm, and
- Providing (104) the analysis information.

2. Method according to claim 1, wherein the reconstruction algorithm comprises an multi-channel reconstruction algorithm and/or an artifact reduction algorithm, wherein the step of applying the analysis algorithm (103) comprises generating multi-channel reconstructed image data by applying the multi-channel reconstruction algorithm to the image data and/or generating artifact reduced image data by applying the artifact reduction algorithm to the image data, wherein reconstructed image data comprise and/or are based on the multi-channel reconstructed image data and/or the artifact reduced image data.

3. Method according to claim 1 or 2, **characterized in** an auto contouring step (105), wherein the auto contouring step (105) comprises generating auto contoured image data by applying an auto contouring algorithm to the reconstructed image data, wherein the analysis input data comprise the auto contoured image data, wherein the analysis algorithm is configured to detect the overdosing based on the auto contoured image data.

4. Method according one of the previous claims, **characterized in** a derivative image data step (106), wherein the derivative image data step (106) comprises generating derivative image data by applying a derivative image data algorithm to the reconstructed image data, wherein the analysis input data comprise the derivative image data, wherein the analysis algorithm is configured to detect the overdosing based on the derivative image data.

5. Method according to claim 4, wherein the derivative image data algorithm is configured to generate a derivative map, a water/Ca map, a CS/PE map and/or a mono-energetic map based on the reconstructed image data, wherein the derivative image data comprise the derivative map, water/Ca map, CS/PE map and/or a mono-energetic map.

6. Method according to one of the previous claims, **characterized in** a radiomic analysis step (107), wherein the radiomic analysis step (107) comprises generating radiomic analysis data by applying an radiomic algorithm to the image data, the reconstructed image data, the auto contoured image data and/or the derivative image data, wherein the analysis input data comprise the radiomic analysis data, wherein the analysis algorithm is configured to detect the overdosing based on the radiomic analysis data.

7. Method according to one of the previous claims, wherein the analysis algorithm is configured to detect overdosing based on a contrast and/or contrast between tissues and/or structures.

8. Method according to one of the previous claims, **characterized in** a attenuation coefficient step (108), wherein the attenuation coefficient step (108) comprises generating attenuation coefficients by applying an attenuation coefficient determination algorithm to the image data, the reconstructed image data, the auto contoured image data and/or the derivative image data, wherein the analysis input data comprise the attenuation coefficients, wherein the analysis algorithm is configured to detect the overdosing based on the attenuation coefficients.

9. Method according to one of the previous claims, wherein in step of acquiring the image data (101) comprises acquiring image data based on photon counting computed tomography.

10. Method according to one of the previous claims, wherein the step providing the analysis information (104) comprises providing the analysis information to a treatment planning algorithm, wherein the treatment planning algorithm is configured to adapt a treatment plan based on the analysis information, wherein the treatment plan is provided to a radiotherapy device (206) to deliver the radiotherapy according to the provided treatment plan.

11. A System (200) for determining an overdosing in radiation therapy, comprising:
- a photon counting imaging device (201) configured to acquire image data,
- a reconstruction module (203) configured to apply a reconstruction algorithm to the image data provided by the photon counting imaging device (201) and generate reconstructed image data,
- an analysis module (204) configured to apply an analysis algorithm to analysis input data and generate analysis data, wherein the analysis input data comprise the reconstructed image data, wherein the analysis algorithm is configured to detect an overdosing based on the analysis input data, wherein the analysis data comprise the detected overdosing and/or an information about the overdosing detected by the analysis algorithm, and
- a providing module (205) configured to provide the analysis information.

12. The system (200) according to claim 11, wherein the photon counting imaging device (201) is configured as a photon counting cone beam computed tomography (PC CBCT) device and/or comprises a flat panel photon counting detector.

13. The system (200) according to claim 11, further comprising a radiotherapy treatment unit (206) configured to deliver radiation therapy to a patient (202) based on the analysis data.

14. The system (200) according to claim 13, wherein the radiotherapy treatment unit (206) comprises a linear accelerator configured to generate high-energy X-rays for the radiation therapy.
